# EUROPEAN PATENT SPECIFICATION

(11) **EP 1 682 213 B1**
(45) Date of publication and mention of the grant of the patent: **16.11.2011**
(21) Application number: 04785297.5
(22) Date of filing: 28.09.2004
(51) Int. Cl.: A61N 5/10

(54) **APPLICATOR FOR RADIATION TREATMENT OF A BODY CAVITY**
APPLIKATOR FÜR STRAHLENBEHANDLUNG EINES HOHLORGANS
APPLICATEUR POUR UN TRAITEMENT DE CAVITE PAR RAYONNEMENT

(30) Priority: 10.10.2003 US 683885
(43) Date of publication of application: 26.07.2006
(73) Proprietor: Xoft Microtube, Inc., Fremont, CA 94538 (US)
(72) Inventor: STEWART, Daren, L., Belmont, CA 94002 (US); LOVOI, Paul, A., Saratoga, CA 95070 (US); RUSCH, Thomas, W., Hopkins, MN 55305 (US); LIM, Alex, Santa Clara, CA 95051 (US); FRANCESCATTI, Darius, Barrington, IL 60010 (US)
(74) Representative: Meyer, Ludgerus
(86) International application number: PCT/US2004/032092
(87) International publication number: WO 2005/037363

(56) References cited:
- WO-A-93/04735
- WO-A-96/40347
- WO-A-97/18012
- WO-A-2004/043531
- US-A- 5 423 745
- US-A- 5 702 368
- US-A1- 2003 032 851
- US-A1- 2004 087 827
- US-B1- 6 398 752

## Description

### Background of the Invention

This invention concerns an applicator for treatment, particular radiation treatment, of a body cavity. More specifically, the invention is useful for radiation treatment of a cavity following surgical resection of a tumor, especially a breast tumor.

Treatment of surgical cavities, such as after malignant tumor excision, has been accomplished with applicators which are inserted usually into a newly formed opening through the skin, a conveniently located opening into the surgical resection cavity. Generally the location is different from the surgical closure itself. Proxima Therapeutics, in Patents Nos. 5,913,813, 5,931,774, 6,083,148, 6,413,204 and 6,482,142, has disclosed applicators which essentially comprise a balloon of known and relatively rigid geometry, i.e. spherical, expandable generally from about four to six centimeters, i.e. designed to have an inflated size of about four to six centimeters diameter. The prior art was limited to the use of such known-geometry balloons that were inflated with a liquid and in which an applicator guide would be positioned, to receive a radiation source.

In the prior art, the applicator guide extended straight out from the insertion wound, and generally the tube was folded down and dressed following an initial treatment, requiring removal of dressing and re-dressing with every subsequent radiation treatment, often twice per day. Such tube handling is satisfactory for a surgical drain, since the drain tube need not be prepared for additional treatments, but is generally unsatisfactory for a radiation procedure involving repeated treatments.

With balloons limited to known geometries, there are limitations in the ability to treat a cavity margin thoroughly. In some cases, the patient cannot take advantage of such a treatment protocol because the known-geometry balloon applicator simply cannot fill many surgical cavities that are irregular in shape. Other measures have to be used in those cases, such as external radiation therapy.

Insertion of such prior balloon applicators has also presented some problems. The balloons, usually of silicone material, encounter friction on insertion through the wound made for this purpose, making insertion difficult, possibly causing unneeded patient trauma and preventing correct positioning of the balloon in the cavity.

Another important consideration in radiation treatment inside an excision cavity is the need to confirm balloon position and position against the cavity wall prior to treatment. Typically physicians add a contrast medium to the balloon inflation liquid, to make the balloon visible by x-ray. The concentration of medium may be inconsistent, however, affecting dose during treatment. A better means of introducing x-ray contrast is needed.

### Summary of the Invention

An applicator according to the invention is provided in claim 1.

The invention disclosed herein improves applicators in a number of ways. The applicator allows for superior wound closure management, with integrated drains and wound closure devices and providing that wound dressing need not be changed each time a radiation therapy device is inserted into the applicator. Basically, the applicator of the invention has an extending tube which can bend without disturbing the dressing and the antiseptic nature of the dressing. A strain reliever preferably is attached or is apart of the tube, and when the tube is inactive a holder device can be incorporated in the wound closure apparatus to hold the tube in an inactive position against the skin.

In addition, either as a part of the applicator itself or as another device integrated with the wound closure element, a drain can be incorporated to bring fluid to the exterior of the body, through another tube or integrally through the main shaft of the applicator, which preferably has several lumens or channels.

Insertion of the balloon or applicator of the invention is accomplished using an obturator (a rod-like device), as in the prior art, which is effective to push the deflated applicator fully into the surgical cavity. One aspect of the invention, however, is that the balloon is coated with a "slippery coat" so that it easily passes through the insertion opening and into the excision cavity without excessive resistance, friction and discomfort.

Another important consideration is the manner in which the applicator is shaped to the cavity. In some embodiments rather than having a prescribed-geometry balloon, the applicator is made to be highly conforming to irregularly-shaped cavities. The geometry of the cavity and applicator, once installed and inflated, can then be determined by self mapping techniques as described in copending application Serial No. 10/464,140, filed June 18, 2003, or external imaging can be performed, provided the applicator has contrast markers. In this way, virtually any shape of excision cavity can be properly treated, without gaps between the applicator and the cavity wall.

Multiple applicators, e.g. multiple balloons, can be aggregated in an application of a further embodiment of the invention. The advantage of having a multiple radiation source applicator and treatment system is that irregular surgical cavities can be treated, especially in conjunction with internal and external radiation detectors as described in copending application Serial No. 10/464,140 referenced above. To take advantage of the irregular nature of some surgical cavities the source guides need to comply with the cavity geometry. With the use of a single balloon, the stretch between radially positioned x-ray guides constrains how far each guide can comply from the adjacent guide. This compliance may not be sufficient to match the irregular cavity. A method of achieving the necessary compliance is to use a separate balloon for each guide. The balloons can be joined to the central lumen but be independent of the neighboring balloons. This allows one guide to comply without constraint from neighboring balloons. The collection of balloons, if they had no circumferential pressure on one another, would tend to move together and not stay circumferentially equally spaced. The bulge in each balloon will press against its neighbor, forcing nearly equal circumferential spacing but without the source guide to source guide tension found in a single balloon design.

Another embodiment of the invention has an applicator that facilitates variation in the radial distance of the x-ray source from the central shaft. Multiple x-ray source applicators have previously been designed to have the guides in direct contact with the balloon-wound interface. It would be an advantage to allow the guides to be a variable radial distance from the central shaft. This will allow the guides to be either compacted around the central inter lumen, expanded out to the wall of an outer balloon or set anywhere in between. An example where this could be used to advantage would be to provide one dose of radiation with the guides near the center of the balloon, in one, several or all of the guides, another dose of radiation with the guides at some intermediate distance from the center of the balloon, again with one, several or all of the guides used which may be different from the first set of guides used, and another dose of radiation with the guides expanded to the outer balloon. In this way a tailored isodose curve can be achieved with very special variations for restricting the dose to specific structures or increasing the radiation dose to other specific structures or volumes.

In another aspect of the invention, a bio-erodible applicator, not in the form of an inflatable balloon, is used to expand the excision cavity. The applicator may be in the form of a basket or helical wire device, a portion of which can be rotated to deploy in the cavity to fully expand against the cavity walls. Here, contact at every point of the wall is not as critical, although the applicator should contact essentially all of the cavity wall areas so as to provide for mapping of the shape of the cavity for development of the treatment plan. Erodible materials, which are further described below are fully absorbed into the body at a time after the series of treatments is completed.

Several polymers based upon bio-compatible plastics as described in detail below dissolve when exposed to body fluids. That is, they basically dissolve over time and are absorbed by the body. These materials can be used to form devices that have a function initially and are left in the body to avoid the effort, inconvenience or trauma of removing the device from the body. A basket or weave made from such material could be used to hold the cavity open and provide a guide for the x-ray source during treatment and then left in place to hold open the wound for a short period and then dissolve away allowing the resection cavity to collapse and heal naturally. The pig tail that emerges from the breast for guiding the x-ray source into the applicator could be cut off, the entry wound dressed and closed allowing the remaining device to dissolve as discussed above. These plastic-like materials tend to be fairly rigid so thin sections or fibers could be used together, like fiber optics made from rigid glass, that would be flexible and strong. This basket could unfold under rotation or an insertion device such as a balloon could be used to expand the basket to fill the cavity. Besides the advantage of not having to remove the applicator there are other advantages to a basket approach. The expansion of a basket applicator would not be constrained by tension between ribs of the applicator which prevents single conventional balloons from filing irregular cavity shapes. There would be little or no lateral tension in the woven basket approach so each rib could comply with the cavity wall independently of neighboring ribs.

Another advantage of the bio-erodible design is the elimination of concern over adhesion. The applicator is placed in the cavity typically for up to two weeks. A conventional applicator may form adhesions between the applicator and the healing wound making removal difficult or in some cases impossible without surgical assistance. With a bioerodible structure adhesion is a moot issue since the structure that is being adhered to vanishes and the structure does not need to be removed in the first place.

Further, the bio-erodible material of the applicator can be impregnated with drugs to deliver a sustained-released drug to the wound, for wound healing, for pain management or other purposes, such treatments themselves being well known in the art.

The most common matrix materials for drug delivery have typically been polymers. The field of biodegradable polymers has developed rapidly since the synthesis and biodegradability of polylactic acid was reported by Kulkarni et al., in 1966 ("Polylactic acid for surgical implants," Arch. Surg., 93:839). Examples of other polymers which have been reported as useful as a matrix material for delivery devices include polyanhydrides, polyesters such as polyglycolides and polylactide-co-glycolides, polyamino acids such as polylysine, polymers and copolymers of polyethylene oxide, acrylic terminated polyethylene oxide, polyamides, polyurethanes, polyorthoesters, polyacrylonitriles, and polyphosphazenes. See, for example, U.S. Pat. Nos. 4,891,225 and 4,906,474 to Langer (polyanhydrides), U.S. Pat. No. 4,767,628 to Hutchinson (polylactide, polylactide-co-glycolide acid), and U.S. Pat. No. 4,530,840 to Tice, et al. (polylactide, polyglycolide, and copolymers).

Degradable materials of biological origin are well known, for example, crosslinked gelatin. Hyaluronic acid has been crosslinked and used as a degradable swelling polymer for biomedical applications (U.S. Pat. No. 4,957,744 to Della Valle et al.; (1991) "Surface modification of polymeric biomaterials for reduced thrombogenicity," Polym. Mater. Sci. Eng., 62:731-735!).

In Patent No. 5,747,058 is disclosed a composition for controlled release of substances including a non-polymeric, non-water-soluble high-viscosity liquid carrier material of viscosity of at least 5.000 cP at 37° C that does not crystalize neat under ambient or physiological conditions; and the substance to be delivered. The patent describes biodegradable compositions that can be used with a bio-erodible applicator device of the invention.

It is therefore among the objects of the invention to provide an improved applicator, particularly for brachytherapy following a breast tumor excision, with improved ease of use, reliability and applicability for virtually any cavity shape, and other enhanced functions. These and other objects, advantages and features of the invention will be apparent from the following description of preferred embodiments, considered along with the drawings.

### Description of the Drawings

Fig. 1 is a system drawing showing components of a post-surgery applicator device, as used on a breast and including a controller.
Fig. 2 is a perspective view schematically showing an applicator of the invention, with sealing devices to seal against the patient's skin.
Figs. 3 and 3A are views showing the device of Fig. 2 as used on a patient and showing two positions of the exterior portion of the device.
Fig. 4 is another view of an applicator according to the invention, with depth indicator markings to assist in proper insertion.
Figs. 5 and 6 are sectional and elevation views showing examples of balloon shape as defined by variable wall thickness.
Figs. 7, 8 and 9 are views showing the use of ribs on a balloon to control the shape of the balloon as inflated.
Figs. 10 and 10A show an embodiment of an applicator in which multiple balloons are bonded together, one inside the other, and with an x-ray absorbent contrast medium in an unbonded area between them.
Fig. 11 shows another applicator embodiment in which a balloon has varied wall thickness with an embedded contrast medium which has greater effect in thicker regions.
Figs. 12 and 13 are end-looking sectional views of modified forms of applicators having radially-disposed multiple balloons connected to a central inflation lumen.
Figs. 14 - 14C are views showing an applicator having an expansion device which is not a balloon, showing stored position and positions progressing to deployment.
Fig. 15 is an end view of the device of Fig. 14.
Fig. 16 is a transverse cross-sectional schematic view showing another form of applicator, with inner and outer balloons and guides carried on the inner balloon.
Figs. 17, 18 and 19 are side sectional schematic views of the Fig. 16 device, showing the inner balloons at different degrees of inflation.
Fig. 20 is a graph showing x-ray absorption for imaging a balloon having integral contrast medium.
Fig. 20A is a graph showing path length data related to Fig. 20.
Fig. 21 is a schematic side cross-section of a patient breast and showing an applicator as in Fig. 20 disposed in a cavity of the breast, indicating an x-ray image of the applicator in the breast cavity.
Fig. 22 is a sectional schematic view showing a stiffening device for a central lumen of a balloon applicator.
Fig. 23 is a schematic view, partly in section, showing an applicator with a drain system for draining a seroma and other liquids from a wound.
Fig. 24 is an end view of the applicator of Fig. 23.
Fig. 25 is a schematic perspective view showing in detail a portion of the applicator of Fig. 23.
Figs. 26-30 are schematic representations of balloon applicators having external devices for promoting drainage from the surgical cavity.

### Description of Preferred Embodiments

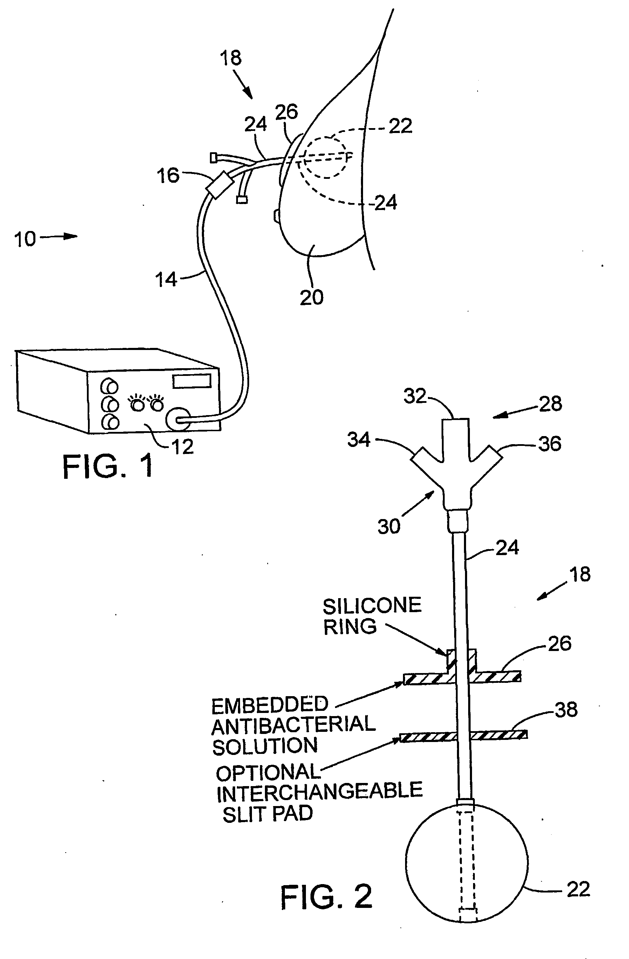

Fig. 1 shows an apparatus 10 according to one embodiment of the invention, including a controller 12, a flexible control line 14, a connection 16 and an applicator generally identified as 18. The applicator 18 is shown as inserted into a breast 20, with an applicator balloon 22 inflated inside a cavity, i.e. a post-surgery excision cavity in the breast. The balloon is supported by a flexible shaft 24 which extends through an exit opening in the breast and through the skin, with the shaft being sealed against the surface of the breast by a seal 26.

Fig. 2 shows the applicator 18 in greater detail, with the balloon 22 shown inflated. At the proximal end 28 of the applicator is a branch 30. The three ports 32, 34 and 36 of this branch device may comprise a service port, a drainage port and a balloon inflation port, respectively. The functions of these ports are further explained below with reference to other drawings. Fig. 2 also shows the seal 26, which preferably is closely and slidably fitted over the exterior surface of the flexible main shaft 24, and which may comprise a silicone ring, or another suitable flexible elastomer. The silicone seal may include an embedded antibacterial solution to help prevent infection from occurring at the wound opening. Optionally, an interchangeable seal pad 38 may be used rather than the seal 26. This pad is also an annular seal with a central hole slidable on the shaft 24, but including a radial slit from the central hole outward to allow interchangeability of the pad.

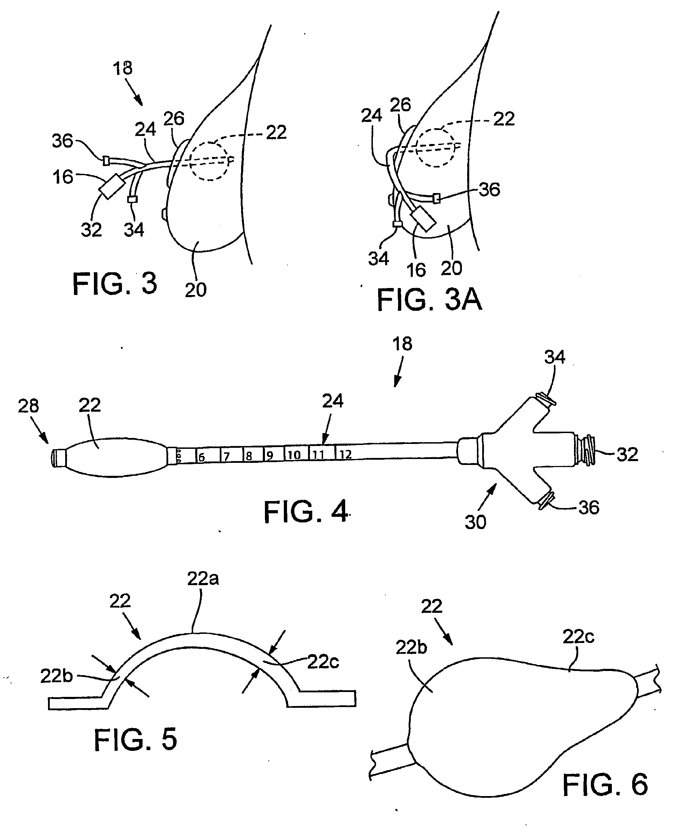

The shaft 24 is flexible, and in particular, it is highly flexible and pliable near the proximal end 28. This facilitates the ability to fold or rather sharply bend down the flexible shaft 24 where it exits the breast, as shown in Figs. 3 and 3A. A strain reliever (not shown), comprising tapered elastomeric sleeve, can be used over the bending region to prevent prolapsing. As explained above, the flexible shaft provides a lumen for admitting a fluid to inflate a balloon, while also providing a duct or lumen for insertion of one or more radiation sources, to enter guides connected to the balloon. The shaft 24, as also explained above, preferably provides a channel for drainage of liquids from the cavity. Instead of intermittently disturbing a taped and dressed wound closure to bring an applicator lumen or shaft into position for repeated radiation treatments, requiring re-dressing, re-taping and closing each occurrence, the invention with the pliable proximal shaft portion 24 allows a comfortable folding down of the shaft end from the position in Fig. 3 to the position in Fig. 3A, without disturbing the seal 26, and while still allowing the shaft its drainage function. A drainage receptacle can be connected to the end of the drainage port or an aspirator can be used when needed to withdraw liquids.

Fig. 4 shows the applicator 18 of a preferred embodiment of the invention with the balloon 22 collapsed. The service port 32, in line with the flexible shaft 24, as well as the drainage port 34 and the balloon inflation port 36, are illustrated. Also shown is a distance scale preferably included, with distances shown at 6 cm, 7 cm, 8 cm, etc., up to about 15 cm, to indicate to the physician the total depth of the applicator into the cavity and opening wound. This provides a direct and easily used means to determine the position of the distal end 29 of the applicator as it is being inserted.

Although not capable of illustration, the applicator 18 preferably is inserted with a "slippery coat" covering the balloon and flexible shaft 24 as they are inserted according to the method of the invention. Such material has typically been used in other procedures, such as for coronary catheters.

The balloon 22 preferably is made of a silicone material, generally as disclosed in some of the Proxima Therapeutics patents noted above, although other appropriate biocompatible materials can be used. It is bonded to the outside surface of the flexible shaft 24 in sealed relationship thereto, by known procedures.

Figs. 5 and 6 illustrate one aspect of the invention whereby the shape of the balloon 22 can be controlled by using varied thickness in the balloon wall 22a. Fig. 5 shows that the balloon may have a normal thickness as shown at the left side of the drawing, at a region 22b, e.g. in an approximate thickness range of about .015 inch to .025 inch. At a region 22c toward the right, however, the thickness is greater, and may be in a range, for example, of about .025 inch to .05 inch. The effect of this is shown approximately in Fig. 6. On inflation the balloon 22 assumes not a spherical shape but approximately a pear shape, because the thicker-walled region 22c is less flexible for expansion and is unable to inflate to the extent of the thinner-walled region 22b.

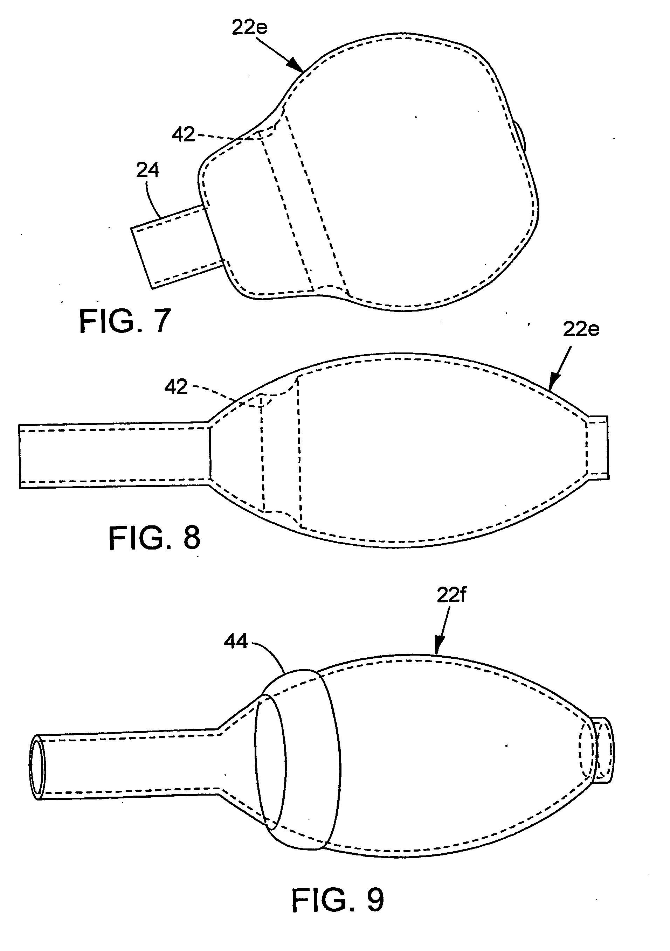

Figs. 7, 8 and 9 illustrate the use of ribs on a balloon to control the inflated shape. In Figs. 7 and 8 an internal rib 42 is shown on a balloon 22e, Fig. 8 showing the balloon essentially unstretched and Fig. 7 showing the balloon inflated and stretched. The rib 42 puts a constraint on the girth of the balloon at the location of the rib, again causing the balloon to assume generally a pear shape. The thickness of such a rib 42 can be adjusted to adjust the effect on the inflated balloon, with a thicker rib having more constraining influence on that area of the balloon.

Fig. 9 shows an essentially unstretched balloon 22f with an exterior rib 44, for the same purpose. It should be understood that the ribs 42 or 44 in these drawings could be several in number and could be positioned anywhere on the balloon, to create the desired shape. These shapes are useful for resection cavities of unusual shape, helping to assure that the balloon walls can be inflated essentially into full contact with the walls of the cavity.

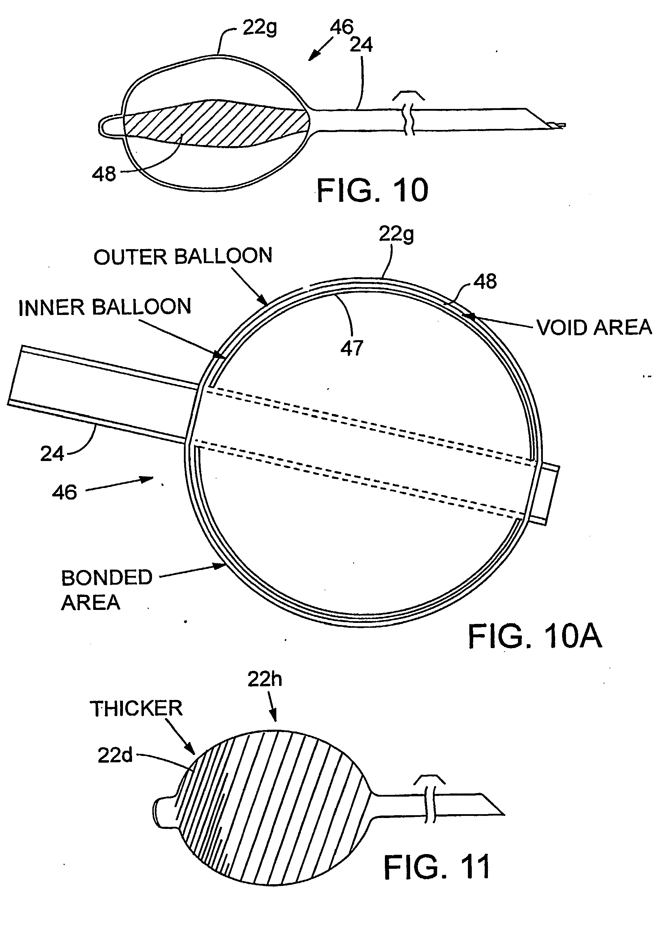

Figs. 10 - 10A show another embodiment of an applicator 46, in this case comprising a balloon 22g which contains another balloon 47 inside in coaxial relationship and essentially in contact. The balloons are secured to a flexible shaft 24. These inner and outer balloons are bonded together around their interface, except at a selected region shown at 48. This provides a space at the region 48, where a contrast medium can be inserted so as to control the radiation delivered to the cavity. Guides for radiation sources, either isotopes or switchable miniature x-ray tubes, are included in the inner balloon, not shown in Figs. 10 and 10A. These guides position the x-ray sources at desired locations in each balloon, and the contrast medium shown at 48 (Fig. 10) can be oriented to a desired position to limit radiation to part of the cavity as needed. The region 48 can be as large or small as needed and in any desired shape and location. The contrast medium can be injected between the layers through the outer balloon, via a lumen in the flexible shaft 24.

In Fig. 11 a balloon 22h is shown inflated and with different wall thicknesses. In a region 22d near the distal end of the applicator, the wall thickness is greater, while being less in the remainder of the balloon. In this case the shape is modified somewhat by the wall is only slightly thicker'and the shape modification is minimal. The thickness variation here is primarily for the purpose of increasing the effect of a contrast medium embedded into the balloon wall material. Where the wall material is thicker, as at 22d, the radiation will be blocked to a much greater extent than where the wall is thinner. Thus, isodose profile can be controlled in this way.

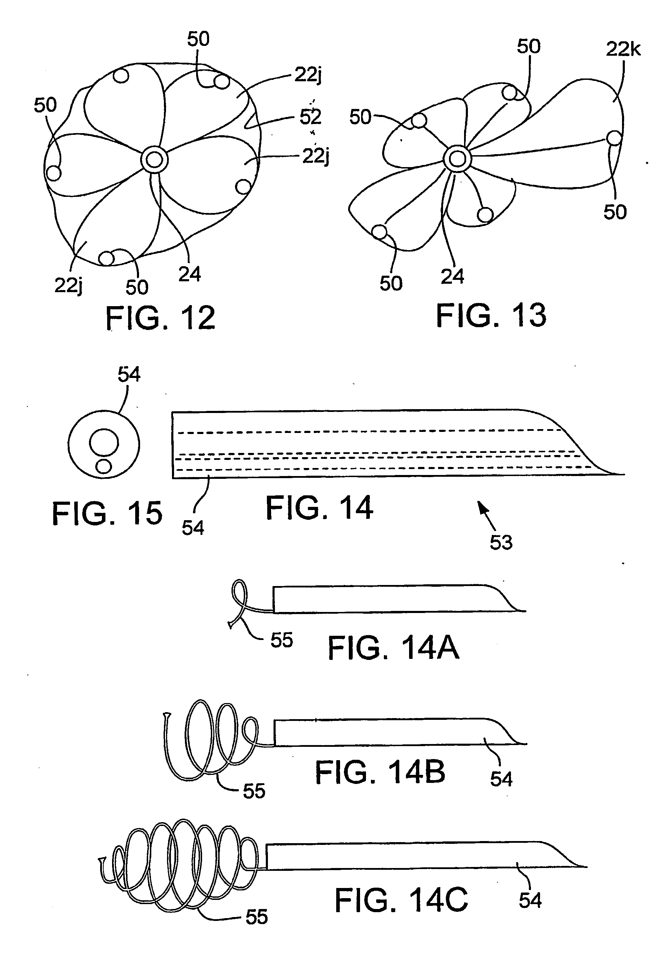

Figs. 12 and 13 show an embodiment of the invention wherein an applicator has multiple balloons arranged radially around a flexible shaft. This can help to locate guides for radiation sources fully adjacent to the cavity wall without the constraints present in a single balloon. In Fig. 12, which is a schematic view in cross section, five separate balloons 22j are shown radially disposed and connected to a generally central flexible shaft 24. The number of balloons can vary. Each balloon in this example has a guide 50 essentially at its outer periphery as shown. The generally central flexible shaft 24 can have separate individual lumens leading to each balloon if desired for individual inflation control of the balloons, or they can be on a common inflation lumen, each thus being inflated to the same pressure but still tending to settle the entire array of balloons into a proper positioning in the cavity 52 as the balloons are inflated, with the cavity confining some balloons and thus causing other balloons to assume a larger volume to fill regular spaces in the cavity.

Fig. 13 shows an example similar to Fig. 12, wherein a balloon 22k is actually of a larger radial length than the other balloons, each of which varies somewhat in radial length from the others. In this case the balloon 22k can be of larger initial (collapsed) size, or the drawing can be considered to illustrate what occurs in a unusually shaped resection cavity, with the radial array of balloons assuming the shape of the cavity as the balloons are inflated.

Figs. 14 to 14C and 15 show one alternative form of applicator 53, not a balloon but rather a basket-like structure which can be expanded. Fig. 14 shows a housing or catheter 53 containing the unexpanded applicator device, essentially a Nitinol wire with shape memory. The catheter 54 is not to scale, shown shortened in these views. An end view of the catheter, with the applicator device undeployed, is shown in Fig. 15. Figs. 14A - 14C show progressively the deployment of the applicator device 55. The wire 55, with shape memory, is pushed out of the catheter 54, and assumes its memorized shape as shown. In Fig. 14C the applicator device is shown fully deployed, with a shape designed to conform to a cavity.

In another embodiment an applicator formed as a basket or frame can be formed of the materials described above, particularly the bio-erodible materials with the advantages described. Moreover, as also described in some detail above, the applicators of Figs. 14 - 15 or other embodiments can be impregnated or coated with a matrix carrying drugs, the matrix itself being biodegradable. These drugs can be for wound healing, for pain management or other purposes, and their use is discussed above. Further in the Fig. 14 embodiment an outer sleeve of bio-erodible material can be deployed and expanded by the Nitinol frame, then the frame removed.

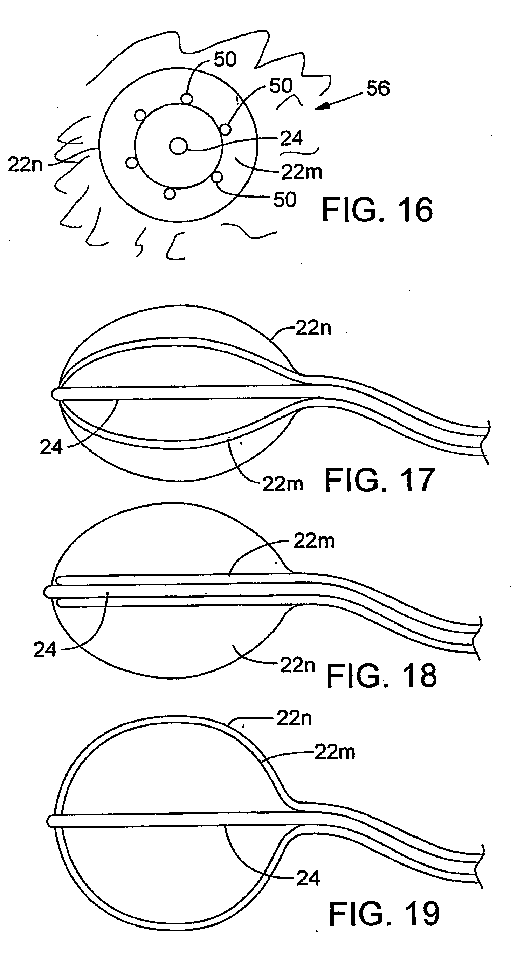

Figs. 16-19 show another embodiment of an applicator 56, in this case comprising an inner balloon 22m within an outer balloon 22n. This arrangement of a balloon inside a balloon, allows a high degree of control of the positions of source guides 50 shown in the sectional schematic view of Fig. 16. The flexible shaft is shown at 24, within the inner balloon 22m. Separate lumens (not specifically shown) are provided in the shaft 24 to feed inflation fluid into the respective inner and outer balloons. The outer balloon will typically be fully inflated to engage against the wall of the cavity. The inner balloon 22m, however, is inflated to varying degrees as needed for the particular procedure, especially to control isodose profile. Fig. 18 shows no deployment of the inner balloon 22m, that is, the inner balloon is collapsed against the shaft 24. The outer balloon 22n is essentially fully inflated. This position puts the guides 50 all closely adjacent to the generally central flexible shaft 24, and this can be a position for administering radiation therapy for a particular case, or it can be important for calibration, as described in the above referenced copending application Serial No. 10/464,140.

A position of medium inner balloon deployment is shown in Fig. 17, and is also indicated in Fig. 16. Again, this position can be useful for certain dose profile requirements. It should be understood that in a medium position of inner balloon deployment, or any position of partial or full inflation of the inner balloon, the guides 50 can be used in a discriminating way such that, if needed to achieve the desired dose, only one or several of the guides can be active in that an isotope or one or more switchable x-ray tubes will be delivering radiation therefrom at any particular time. Also, some or all of the guide positions can be used to deliver radiation at several different incremental degrees of inner balloon inflation, again to achieve the desired isodose profile. The inner/outer balloon arrangement provides a great flexibility for the manipulation of radiation dose by varying the position from which radiation is delivered from one, several or all of the guides mounted on the inner balloon. Fig. 19 shows a position of maximum inner balloon deployment, with the inner balloon 22m substantially engaging against the outer balloon 22n.

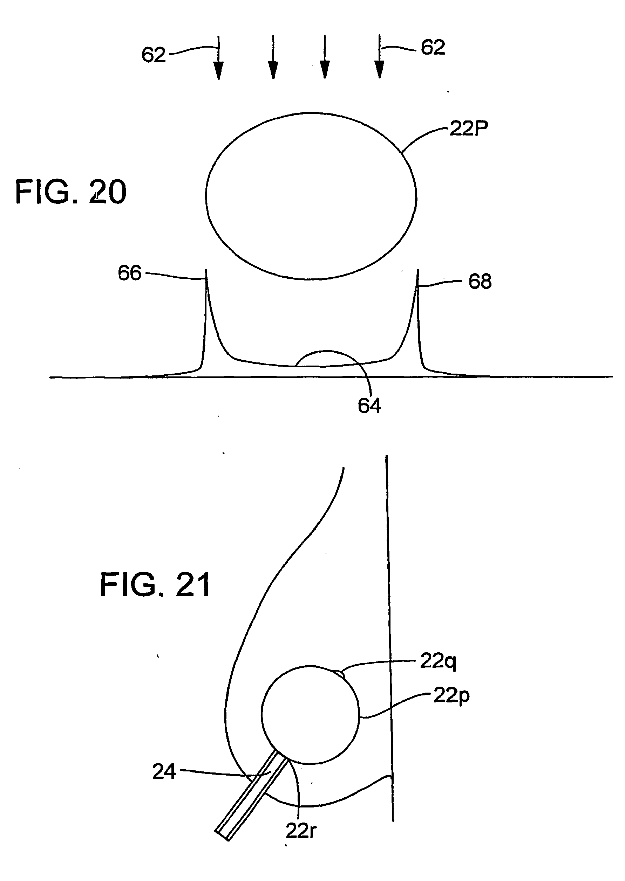

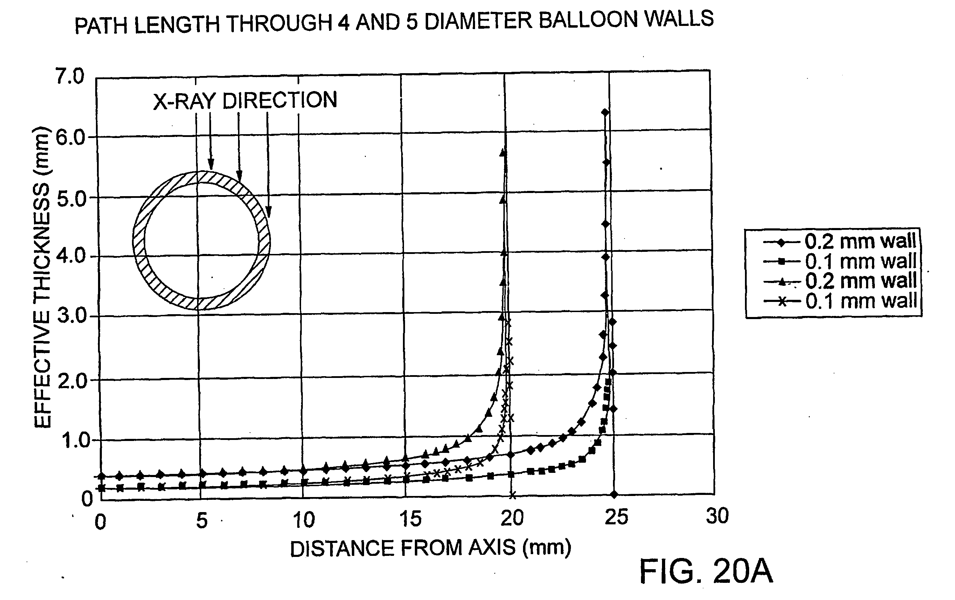

Figs. 20, 20A and 21 illustrate a balloon 22p which has a contrast medium in or on the balloon wall. This contrast medium, as discussed above, will absorb radiation and thus attenuate the radiation delivered from inside the balloon to some extent. However, with a low concentration of such contrast medium in the balloon, the attenuating effect of the medium for radiation passing through the balloon at an angle normal or generally normal to the balloon wall will be small and nearly negligible. However, the effect of radiation, particularly x-ray radiation, passing tangentially through the edges of the balloon, which are what is seen in Fig. 21, will be at maximum, since the radiation must pass through the balloon essentially edgewise at this tangential angle, a much longer effective path length. The result is that a balloon with such contrast medium can be located by external x-ray, visible in an x-ray by its edges as shown in Fig. 21. The darkest outline of the balloon will be at its circumference appearing as in Fig. 21, and especially at distal and proximal ends of the balloon.itself, shown at 22q and 22r, where the wall material may be somewhat thicker at its attachment to the flexible shaft 24 and in any event, where the balloon has areas that are stretched far less due to the geometry of the balloon and its attachment to the flexible guide.

Fig. 20 is a schematic approximation showing a graph of apparent x-ray density (darkness of the line appearing in an x-ray) on the vertical axis, versus position. For clarity a balloon 22p is represented directly adjacent to the graph, and the direction of x-rays which would produce approximately such a graph is shown by arrows at 62. Fig. 20A is a graph of date on effective path length through the balloon versus position, for 4 cm and 5 cm diameter balloons. As illustrated, some density is observed in the middle of the balloon, at a region 64 in Fig. 20 where the radiation passes generally normally through the balloon wall; however, spikes of extreme density are shown at 66 and 68, where the rays must pass through considerable distance of the balloon wall on edge. The effective path length at these tangent regions can be about 15 to 25 times greater than the normal path length.

Fig. 22 shows schematically, and not to scale, an applicator 70 in transverse cross section. The applicator includes a balloon 22s, shown inflated and surrounding a generally central flexible shaft 24. The drawing illustrates the use of a stiffener 72 on that section of the shaft 24 which is within the balloon. Pursuant to the invention the flexible shaft is soft and pliable, particularly at portions designed to be at the exterior of the cavity, and the shaft will usually be one integral extrusion. The pressure of the balloon when inflated can collapse or partially collapse such a soft and pliable shaft. To address this problem, Fig. 22 shows that a stiffening sleeve 22, which may be a polyester sleeve heat shrunk onto the exterior of the shaft 24, can provide the needed stiffness against the tendency to collapse under pressure. The use of a stiffener of this type makes convenient the stiffening of only one section of the flexible shaft, where the balloon surrounds the shaft, without affecting the remaining length of the shaft. Other stiffening devices which could be used are an extrusion over the main extrusion or a separate sleeve over the shaft.

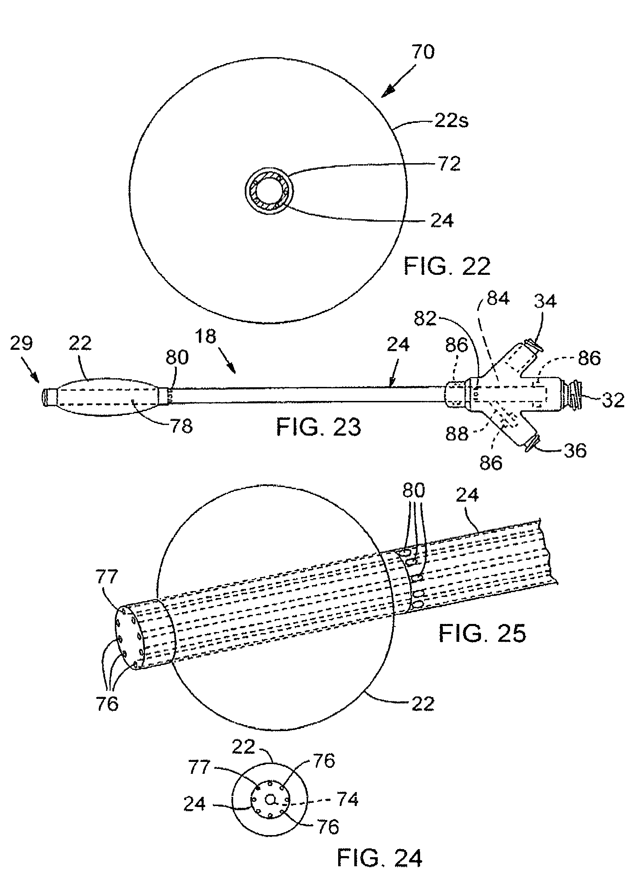

Fig. 23 shows a preferred and specific embodiment of an applicator 18 such as shown in Figs. 1, 2 and 4. Fig. 24 is a distal end view, particularly showing the end of the flexible shaft 24. Fig. 23 shows the branched proximal end 30 of the device, with the service, drainage and balloon inflation ports 32, 34 and 36 respectively. Fig. 25 shows schematically and not to scale a distal portion of the applicator, the portion with the balloon 22, in an enlarged view and with the balloon in cross section. The flexible shaft 24 of the device has a central lumen or main passage 74, surrounded by a series of longitudinal channels 76, 77, of which there may be approximately 5 to 8. The central shaft preferably is formed by extrusion, and can be, for example, silicone, soft polyurethane, or other suitable medical grade materials. The end view of Fig. 24 shows that one of these longitudinal channels or passageways 77 is closed at the distal end. This preferably is to dedicate the channel 77 as an inflation lumen for the balloon 22. For example, a hole 78 may be provided in the wall of the flexible shaft 24, communicating into the inflation channel 77, to provide for admitting an inflating fluid to the balloon.

The remaining channels 76, or some of them, preferably are used for drainage. As shown in Fig. 24, at the distal end of the shaft 24 these channels 76 are shown as open holes, for collecting drainage liquids at the distal end of the cavity. In addition, holes or ports may be formed on the shaft just proximal of the balloon, as shown at 80 in Fig. 23, these ports communicating with the channels 76 within the shaft. Near the proximal end of the applicator are corresponding holes or ports 82 communicating with the same channels. Here, drainage liquid flowing through the channels exits the flexible shaft 24 and enters a plenum 84 surrounding the shaft, formed by the branching device 30. The inflation port 34 has a seal 86, which may be formed by a glue, for example, to prevent escape of drainage liquids through that port. Similarly, a seal 86 is formed at the service port 32 surrounding the end of the flexible shaft 24 (shown in dashed lines here), so that the service port 32 communicates only with the central lumen 74 of the shaft (the drainage channels 76 preferably are blocked off at the proximal end of the shaft, as is the inflation lumen or channel 77, this feature not shown, in the drawings). Thus, the draining liquid can only exit via the drainage port 36.

The inflation port 34 communicates only with the single lumen or channel 77. This is accomplished with a tube 88 which passes through the seal 86 and is sealingly connected to a hole at the exterior surface of the flexible shaft 24, communicating with the appropriate channel within the shaft.

Figs. 24 and 25 show that the distal end of the flexible shaft 24 has the end of the central, large lumen 74 closed off. This larger lumen is for insertion of the radiation sources into the flexible shaft. In the case where the guides are out in the balloon or balloons (e.g. Figs. 12, 13, 16-19), a different shaft or multiple such shafts are used, with guides that extend out into the balloons.

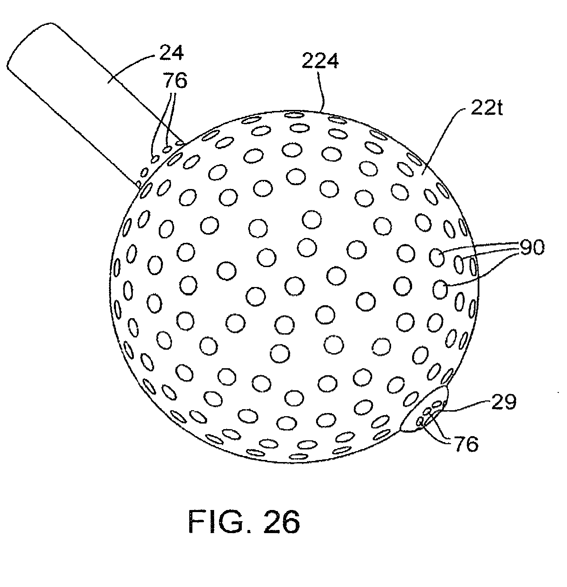

Figs. 26 - 30 show various embodiments of textured balloons on applicators, to enhance drainage of seroma, allowing the liquid to reach the drain holes discussed above. These surface features, essentially bumps, ridges, grooves or interrupted relief lines, are configured to enhance fluid migration. In Fig. 26 a multiplicity of bumps are shown, formed integrally on the exterior surface of a balloon 22t. As noted above, drain holes, communicating with drain channels in the flexible shaft 24, are at 76 and also at the distal end 29 of the shaft. The bumps 90 effectively stand the balloon surface off a slight distance from the cavity wall against which it is engaged, allowing the migration of the liquids.

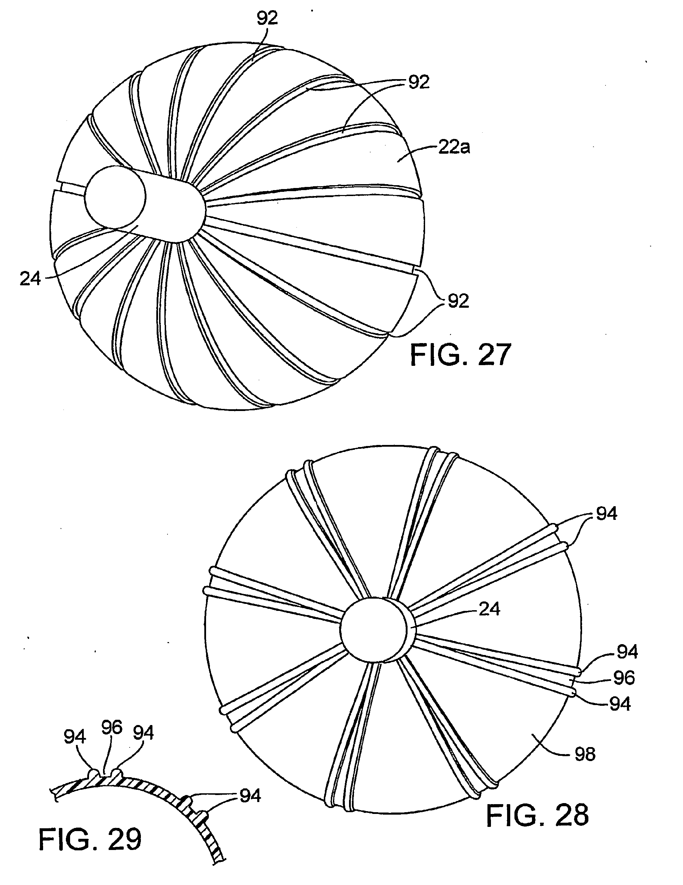

Fig. 27 shows grooves 92 oriented longitudinally on the surface of the balloon 22u, tending to conduct seroma and other liquids in a longitudinal direction for drainage. Figs. 28 and 29 show pairs of ridges 94, forming channels 96 for liquid flow. Wider channels are found between adjacent pairs of ridges, as at 98, but these are wide enough that this area for the most part can engage against the tissue of the cavity wall. The ridges 94 of each pair are sufficiently close together to allow liquid flow, but not to allow tissue to fill the channel.

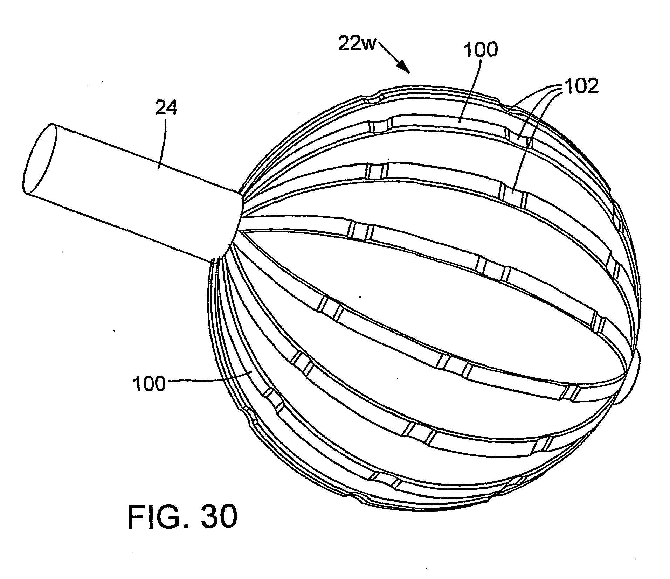

Fig. 30 shows a variation in which ridges 100 on the surface of a balloon 22w are interrupted, with gaps 102 between them. This provides generally for a longitudinal flow of fluid, but allows cross flow across the ridges, particular for gravitational flow of liquid toward the bottom of the cavity.

## Claims

1. An applicator for brachytherapy radiation treatment of a cavity inside living tissue, comprising: a radiation delivery source, an inflatable balloon (22) having a collapsed state and an inflated state and being in a position to contact tissue when inflated in a surgical cavity, a flexible shaft (24) secured to the balloon (22) and being elongated so as to extend from inside the surgical cavity to outside the surgical cavity when installed said flexible shaft (24) having a lumen (77) for introducing a fluid to the balloon (22) to inflate the balloon (22), surface relief means (90, 92, 94) on the exterior of the balloon (22) for providing channels, when the balloon, is inflated, to allow the flow of liquids from the surgical cavity toward the exit of the surgical cavity, and at least one drain channel (76) provided in the flexible shaft (24), positioned to receive draining liquids from the surgical cavity, and means provided in the flexible shaft (24) for conducting said liquids out of the surgical cavity through the drain channel (76), said means comprising entry holes (80) arranged in the flexible shaft (24) proximal of the balloon, and communicating with the at least one drain channel (76), for collecting drain liquids.

2. The applicator of claim 1, wherein the flexible shaft (24) has a central longitudinal channel (24) and a series of outer longitudinal channels (76, 77) arranged generally in an annular array around the central longitudinal channel, at least one of the outer channels comprising said drain channel and being open at a distal end of the flexible shaft to collect liquid.

3. The applicator of claim 2, wherein a proximal end of the flexible shaft (24) is branches, one branch (34) having said drain channel (76) and adapted to an aspirator to draw off liquids, another branch (36) having said lumen (77) for inflation of the balloon, and a further branch (32) having a channel for insertion of a radiation delivering source through said central longitudinal channel (74).

4. The applicator of claim 3, wherein said lumen (77) for inflation of the balloon (22) comprises one of the outer channels.

5. The applicator of claim 3, wherein the balloon (22) includes guides (50) to receive the radiation delivery source, said guides (50) connected to said central longitudinal channel (74) and said further branch (32).

6. The applicator of claim 1, wherein the surface relief means (90, 92, 94) comprises longitudinally extending ridges (94) arranged on the exterior of the balloon (22), providing channels between adjacent ridges.

7. The applicator of claim 6, wherein the ridges (94) are interrupted in their length, providing for cross flow of liquids between channels.

8. The applicator of claim 1, wherein the surface relief means (90, 92, 94) comprises bumps (90) extending outwardly on the exterior of the balloon (22).

9. The applicator of claim 1, wherein the surface relief means (90, 92, 94) comprises grooves (92) extending inwardly on the exterior surface of the balloon (22).

## Patentansprüche

1. Applikator für die Brschytherapie-Strahlenbehandlung eines Hohlraums innerhalb lebenden Gewebes, der Folgendes umfasst: eine Strahlenquelle, einen aufblasbaren Ballon (22), der sowohl einen zusammengefallenen als auch einen aufgeblasenen Zustand haben kann und so positioniert ist, dass er Gewebe kontaktieren kann, wenn er in einem chirurgischen Hohlraum aufgeblasen wird, einen flexiblen Schaft (24), der an dem Ballon (22) befestigt ist und so lang ist, das er sich vom innenraum des chirurgischen Hohlraums nach Außen erstreckt, wenn er installiert ist, wobei der flexible Schaft (24) ein Lumen (77) zum Einführen eines Fluids in den Ballon (22) aufweist, um den Ballon (22) aufzublasen. Oberflächenentlastungsmittel (90, 92, 94) an der Außenseite des Ballons (22) zum Bereitstellen von Kanälen im aufgeblasenen Zustand des Ballons, um den flüssigkeitsfluss vom chirurgischen Hohlraum in Richtung des Ausgangs des chirurgischen Hohlraums zu erlauben, und wenigstens einem in dem flexiblen Schaft (24) angeordneten Drainagekanat (76), der so positioniert ist, dass er Drainageftüssigkeiten aus dem chirurgischen Hohlraum aufnehmen kann, und ein in dem flexiblen Schaft (24) angeordnetes Mittel zum Abführen der Flüssigkeiten aus dem chirurgischen Hohlraum durch den Drainagekanal (76), wobei das Mittel Eintrittslöcher (80) umfasst, die in dem flexiblen Schaft (24) proximal zum Ballon angeordnet sind, und mit dem wenigstens einen Drainagekanal (76) kommunizieren, um abzuleitende Flüssigkeiten aufzufangen.

2. Applikator nach Anspruch 1, bei dem der flexible Schaft (24) einen zentraler langgestreckten Kanal (74) sowie eine Reihe von äußeren langgestreckten Kahälen (76. 77) aufweist, die allgemein in einer kreisförmigen Anordnung um den zentralen langgestreckten Kanal angeordnet sind, wobei wenigstens einer der äußeren Kanäle den Drainagekanal umfasst und an einem distalen Ende offen sit, um Flüssigkeit aufzufangen

3. Applikator nach Anspruch 2, bei dem ein proximales Ende des flexiblen Schafts (24) verzweigt ist und eine Abzweigung (34) den Drainagekanal (76) aufweist und zum Abziehen von Flüssigkeiten an einen Aspirator angepasst ist, eine weitere Abzweigung (36) das Lumen (77) zum Aufblasen des Ballons aufweist, und eine weitere Abzweigunq (32) einen Kanal zum Einführen einer eine Strahlung abgebenden Quelle durch den zentralen langgestreckten Kanal (74) aufweist.

4. Applikator nach Anspruch 3. bei dem das Lumen (77) für das Aufblasen des Ballons (22) einen der äußeren kanäle aufweist.

5. Applikator nach Anspruch 3, bei dem der Ballon (22) Führungen (50) zur Aufnahme der Strahlenquelle umfasst, wobei die Führungen (50) mit dem zentralen langgestreckten Kanal (74) und der weiteren Abzweigung (32) verbunden sind.

6. Applikator nach Anspruch 1, bei dem das Oberflächenentlastungsmittel (90, 92, 94) sich in Längsrichtung erstreckende Rippen (94) umfasst, die an der Außenseite des Ballons (22) angeordnet sind und zwischen einander benachbarten Rippen Kanäle bilden.

7. Applikator nach Anspruch 6. bei dem die Rippen (94) in ihrer Lange unterbrochen sind, um eine Querströmung von Flüssigkeiten zwischen den Kanälen zu erlauben.

8. Applikator nach Anspruch 1, bei dem das Oberfiächenentlastungsmittel (90. 92, 94) Erhebungen (90) umfasst, die sich auf der Außenseite des Ballons (22) nach Außen erstrecken.

9. Applikator nach Anspruch 1, bei dem das oberflächenentlastungsmittel (90. 92, 94) Kerben (92) umfasst, die sich auf der außenseite des Ballons (22) nach Innen erstrecken.

## Revendications

1. Applicateur pour un traitement par rayonnement de brachythérapie d'une cavité à l'intérieur d'un tissu vivant, comprenant : une source d'émission de rayonnement, un ballonnet gonflable (22) présentant un état replié et un état gonflé et étant dans une position permettant d'entrer en contact avec un tissu quand il est gonflé dans une cavité chirurgicale, un arbre flexible (24) fixé au ballonnet (22) et allongé de façon à s'étendre de l'intérieur de la cavité chirurgicale vers l'extérieur de la cavité chirurgicale quand il est installé, ledit arbre flexible (24) comportant une lumière (77) permettant d'introduire un fluide dans le ballonnet (22) pour gonfler le ballonnet (22), des moyens en relief en surface (90, 92, 94) sur l'extérieur du ballonnet (22) pour former des canaux, quand le ballonnet est gonflé permettant l'écoulement de liquides de la cavité chirurgicale vers la sortie de ladite cavité chirurgicale, et au moins un canal de drainage (76) ménagé dans l'arbre flexible (24), positionné de façon à recevoir des liquides de drainage de la cavité chirurgicale, et des moyens ménagés dans l'arbre flexible (24) pour conduire lesdits liquides hors de la cavité chirurgicale, à travers le canal de drainage (76), lesdits moyens comprenant des trous d'entrée (80) dispensés dans l' arbre flexible (24) de manière proximale au ballonnet et communiquant avec ledit canal de drainage (76) pour collecter lesdits liquides de drainage.

2. Applicateur selon la revendication 1, dans lequel l'arbre flexible (24) comporte un canal longitudinal central (74) et une série de canaux longitudinaux extérieurs (76, 77) disposés généralement selon une disposition annulaire autour du canal longitudinal central, au moins un des canaux extérieurs comprenant ledit, canal, de drainage et étant ouvert à une extrémité distale de l'arbre flexible pour collecter du liquide.

3. Applicateur selon la revendication 2, dans lequel une extrémité proximale de l'arbre flexible (24) est ramifiée, une ramification (34) comportant ledit canal de drainage (76) et étant adaptée à un aspirateur pour prélever des liquides, une autre ramification (36) comportant ladite lumière (77) pour le gonflage du ballonnet, et une autre ramification (32) comportant un canal pour l'insertion d'une source d'émission de rayonnement, à travers ledit canal longitudinal central (74).

4. Applicateur selon la revendication 3, dans lequel ladite lumière (77) pour le gonflage du ballonnet (22) comprend un des canaux extérieurs,

5. Applicateur selon la revendication 3, dans lequel le ballonnet (22) comprend des guides (50) pour recevoir la source d'émission de rayonnement, lesdits guides (50) étant: connectés audit canal longitudinal central (74) et à ladite ramification ultérieure (32).

6. Applicateur selon la revendication 1. dans lequel des moyens en relief en surface (90, 92, 94) comprennent des replis s'étendant longitudinalement (94), disposés sur l'extérieur du ballonnet (22), fournissant des canaux entre les replis adjacents.

7. Applicateur selon la revendication 6, dans lequel les replis (94) sont interrompus dans leur longueur, fournissant un flux croisé de liquides entre les canaux.

8. Applicateur selon la revendication 1, dans lequel les moyens en relief en surface (90, 92, 94) comprennent des bosses (90) s'étendant vers l'extérieur sur l'extérieur du balconnet, (22).

9. Applicateur selon la revendication 1, dans lequel les moyens en relief en surface (90, 92, 94) comprennent des cannelures (92) s'étendant vers l'intérieur sur la surface extérieure du ballonnet (22).
